Europäisches Patentamt

European Patent Office

Office européen des brevets

(19)

(11) Numéro de publication : **0 323 322 B1**

(12) **FASCICULE DE BREVET EUROPEEN**

(45) Date de publication du fascicule du brevet :
15.04.92 Bulletin 92/16

(51) Int. Cl.⁵ : **G01N 33/48**

(21) Numéro de dépôt : **88403280.6**

(22) Date de dépôt : **22.12.88**

(54) **Dispositif pour mesurer le temps de modification de l'état physique d'un milieu fluide.**

(30) Priorité : **30.12.87 FR 8718347**

(43) Date de publication de la demande :
**05.07.89 Bulletin 89/27**

(45) Mention de la délivrance du brevet :
**15.04.92 Bulletin 92/16**

(84) Etats contractants désignés :
**AT BE CH DE ES GB GR IT LI LU NL SE**

(56) Documents cités :
**EP-A- 90 192**
**EP-A- 169 794**
**FR-A- 2 383 444**
**FR-A- 2 410 817**
**FR-A- 2 465 227**

(73) Titulaire : **SERBIO**
**30, Avenue Flachat**
**F-92600 Asnières (FR)**

(72) Inventeur : **Martinoli, Jean-Luc**
**2, rue du Tremble**
**F-92390 Villeneuve La Garenne (FR)**
Inventeur : **Rousseau, Alain**
**7, rue Nicolas Houel**
**F-75005 Paris (FR)**
Inventeur : **Vilain, Pascal**
**27, rue Croix du Bellay**
**F-60240 Hadancourt le Haut Clocher (FR)**

(74) Mandataire : **Marquer, Francis et al**
**Cabinet Moutard 35, Avenue Victor Hugo**
**F-78960 Voisins le Bretonneux (FR)**

Jouve, 18, rue Saint-Denis, 75001 PARIS

## Description

La présente invention concerne un dispositif pour mesurer le temps de modification de l'état physique d'un milieu fluide, et, en particulier, pour mesurer le temps de coagulation du sang, du genre comprenant un godet vertical qui contient le fluide, tel que le plasma et les réactifs nécessaires, et dans lequel est placée une bille ferromagnétique entraînée en rotation par un champ magnétique extérieur.

Un tel dispositif est décrit notamment dans le brevet européen N° 90 192, selon lequel la bille ainsi entraînée se déplace librement sur le fond plat et horizontal du godet. Lorsqu'intervient la coagulation du fluide qui est contenu dans ce dernier, et qui est ici du plasma sanguin additionné de réactifs, la bille se déplace sur une courbe dirigée vers l'intérieur à cause, semble-t-il, de l'accroissement de la résistance mécanique opposée par le contenu du godet, et on détecte son déplacement radial par des moyens bien connus en eux-mêmes.

La précision des mesures ainsi réalisées laisse à désirer, parce que le déplacement radial d'une bille vers le centre d'un godet de très faibles dimensions est une grandeur physique qui est difficile à détecter et à évaluer de manière satisfaisante. En outre, la reproductibilité de telles mesures n'est pas bonne, à cause notamment de la complexité des phénomènes qui leur servent de base.

Selon EP A 0 169 794, le godet est entraîné mécaniquement dans un mouvement circulaire non rotatif et le mouvement circulaire résultant de la bille est freiné lors de la coagulation.

Il devient toutefois alors très irrégulier et la détection de ses variations semble être peu précise et semble manquer de fiabilité.

Aussi, le but de la présente invention est de proposer un dispositif pour mesurer le temps de coagulation du sang du genre spécifié ci-avant qui pallie les inconvénients des dispositifs connus et, en particulier, qui permette des mesures dont la précision, la fiabilité et la reproductibilité soient supérieures à celles obtenues jusqu'alors avec des appareils de ce type. D'autre part, un objet de l'invention est un tel dispositif de mesure qui soit pratiquement automatique et dans lequel l'intervention humaine soit réduite au strict minimum, sans que son coût soit prohibitif pour autant.

Ce but et cet objet, ainsi que d'autres qui apparaîtront par la suite, sont atteints selon l'invention grâce à un appareil de mesure comprenant un godet vertical qui contient le fluide et dans lequel sont introduits les réactifs nécessaires, tandis qu'y est placée une bille ferromagnétique entraînée en rotation, et que des moyens sont prévus pour détecter les modifications du mouvement de la bille qui résultent de la coagulation, caractérisé, en combinaison, en ce que la bille est directement entraînée en rotation par un champ magnétique tournant extérieur, que le godet (1) présente à sa partie inférieure un chemin de roulement annulaire (4) pour ladite bille (5), et que les moyens de détection (17) sont agencés pour comparer les vitesses relatives de rotation de la bille et du champ magnétique tournant, au moment de la modification d'état physique.

On comprend que le déplacement de la bille sur un chemin de roulement fixe, et non plus, de manière quasi aléatoire, sur le fond plat d'un godet comme dans le brevet européen N° 90 192 précité, permet une grande précision et une bonne reproductibilité des mesures, puisqu'il suffit de détecter des variations de la 'fréquence de signaux électriques, ce qui est une opération aisée pour les spécialistes en la matière. En outre, cette détection est, de toute évidence, plus facile et moins entachée d'erreurs que celle qui consiste à déterminer l'échappement vers le centre du godet d'une bille de très faibles dimensions.

Selon une forme de réalisation avantageuse du dispositif selon la présente invention, l'entraînement en rotation de la bille ferromagnétique spécifiée plus haut a lieu à partir d'un moteur électrique dont l'arbre de sortie est solidaire d'une tige ferromagnétique qui est disposée sous le godet contenant la bille en ayant le même axe que lui, qui traverse une bobine alimentée en courant et qui se termine à sa partie supérieure par un doigt ferromagnétique excentré par rapport à l'axe commun de cette tige et du godet.

Ce doigt excentré devient un pôle magnétique sous l'effet de la bobine traversée par le noyau qui en est solidaire, et il entraîne en rotation la bille contenue dans le godet avec une vitesse angulaire qui est égale à la sienne propre tant que la coagulation du sang n'a pas ralenti la bille en question. Cette vitesse peut être réglée, comme on le verra plus loin, ce qui procure divers avantages qui seront précisés au moment voulu.

Avantageusement, le dispositif selon l'invention comprend un premier détecteur réflex de type classique associé à la bille en rotation, un second détecteur réflex similaire au premier et associé au doigt excentré qui entraîne la bille, et un comparateur relié à un microprocesseur permettant de repérer tout défaut de synchronisme entre les signaux délivrés par les deux détecteurs ci-dessus. Il suffit alors de comparer les signaux provenant de ces derniers, qui sont identiques entre eux, pour détecter la coagulation du plasma contenu dans le godet. Du fait qu'il s'agit alors d'une comparaison de deux signaux, et non du traitement d'un signal unique, et qu'en outre ces signaux proviennent de deux détecteurs identiques, on augmente la précision de la mesure et on facilite son traitement par des moyens électroniques.

Pour entraîner en rotation la bille ferromagnétique selon l'invention, et selon une variante, le doigt excentré tournant spécifié ci-dessus peut être remplacé par une pluralité de pôles fixes qui entourent le godet contenant la bille, sensiblement sur le même plan que cette dernière, et qui sont magnétisés par des bobines alimentées par ces courants variables de façon à produire un champ tournant dans le plan de la bille.

Quel que soit le mode d'entraînement de la bille, le dispositif de mesure selon l'invention comporte avantageusement des moyens pour imprimer à la bille une vitesse de rotation et une force d'entraînement variables en fonction du test réalisé et de la durée de la mesure.

Selon une forme de réalisation avantageuse, cette vitesse de rotation et cette force d'entraînement sont plus fortes au début de la mesure qu'à la fin, avec, de préférence, un palier au début de la mesure où la vitesse de rotation et la force d'entraînement de la bille sont relativement élevées, et un palier à la fin de la mesure où ces valeurs sont plus faibles, ces deux paliers étant reliés par une diminution progressive et, par exemple, linéaire.

Cette loi de pilotage repose sur le fait que, dans les essais d'hémostase dont il est question ici, des caillots de viscosités très différentes sont créés dans des intervalles de temps qui sont, eux aussi, très différents, les caillots forts apparaissant au début et les caillots faibles à la fin. La régulation selon l'invention de la vitesse de rotation de la bille et de sa force d'entraînement permet de concilier une bonne précision, de l'ordre de 0,1 seconde pour les caillots forts, et une grande sensibilité pour les caillots faibles, la précision s'obtenant avec une vitesse élevée qui peut être de l'ordre de 10 tours par seconde, et la sensibilité avec une force d'entraînement et une vitesse réduites, cette dernière pouvant être égale à 3 tours par seconde environ.

Dans la forme de réalisation spécifiée ci-avant où la bille est entraînée par un doigt tournant excentré, cette régulation est avantageusement obtenue grâce au fait que son moteur d'entraînement est un moteur à courant continu et qu'il est alimenté, ainsi que sa bobine d'aimantation, par l'intermédiaire de moyens de régulation respectifs commandés par un microprocesseur. On règle alors très simplement la vitesse en faisant varier la tension continue aux bornes du moteur, et la force d'entraînement en agissant sur le courant qui traverse la bobine, la loi de variation en fonction du temps de ces deux valeurs étant commandée par le microprocesseur.

Dans une forme de réalisation avantageuse, le dispositif selon la présente invention peut inclure également un densitomètre optique comprenant une diode électroluminescente qui est alimentée à partir d'un microprocesseur et qui éclaire une photodiode à travers un filtre optique passebande, le faisceau lumineux passant à travers le godet de l'appareil de l'invention, au-dessus de la bille qu'il contient, et les informations de la photodiode étant transmises au microprocesseur spécifié ci-avant.

Bien que ce densitomètre destiné aux mesures dites de colorimétrie soit indépendant du coagulomètre mécanique qui vient d'être décrit, l'association de ces deux appareils dans une même unité simplifie notablement un certain nombre d'opérations. Elle permet un outre de diminuer les coûts, du fait que l'on peut utiliser à deux fins différentes divers organes annexes, comme les dispositifs de régulation de la température, les alimentations et/ou une partie au moins des interfaces homme/machine, ces organes pouvant en outre être communs à plusieurs godets de mesure logés dans le même appareil. D'ailleurs, la bille du coagulomètre mécanique peut également servir d'agitateur pour le densitomètre.

La description qui va suivre, et qui ne présente aucun caractère limitatif, permettra de bien comprendre comment la présente invention peut être mise en pratique. Elle doit être lue en regard des dessins annexés, parmi lequels:

– La figure 1 représente une vue en coupe axiale schématique du dispositif de mesure selon l'invention;

– La figure 2 montre un diagramme schématique qui représente les circuits électriques principaux de ce dispositif; et:

– La figure 3 représente deux courbes qui traduisent la loi de pilotage de ce dispositif en fonction du temps.

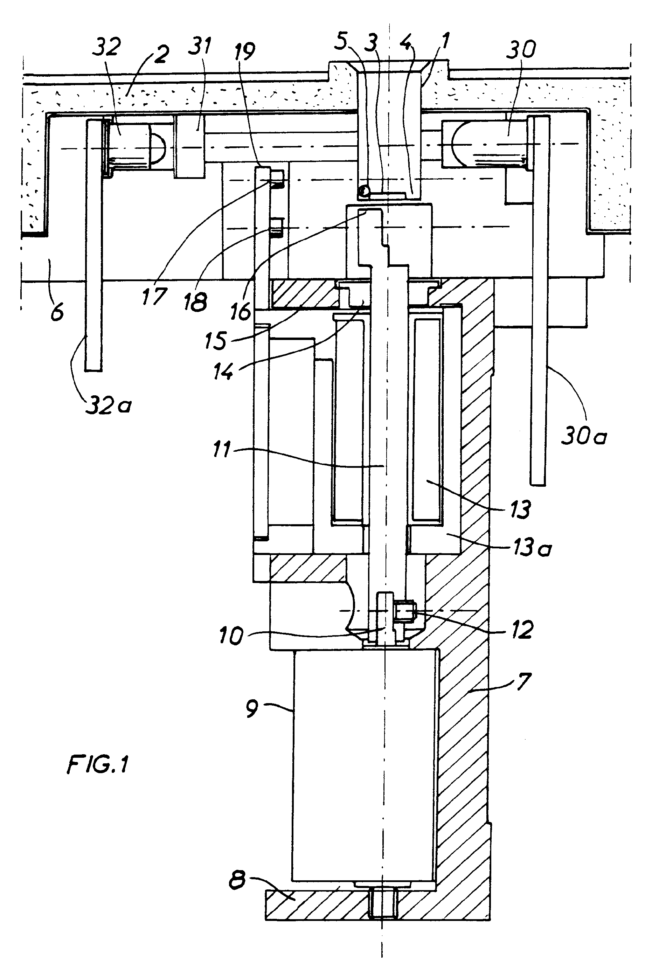

Dans sa forme de réalisation représentée sur la figure 1, l'appareil de mesure selon l'invention comprend un godet cylindrique 1 qui est inséré verticalement à frottement doux dans une platine supérieure 2 et dont le fond comporte un plot central 3, ce qui définit entre ce dernier et la paroi du godet 1 un chemin de roulement annulaire 4 destiné à une bille 5 en une matière ferromagnétique qui est généralement de l'acier.

A la partie inférieure de la platine 1 est vissé un bloc de mesure 6 qui est solidaire d'un bâti inférieur 7 sur la base horizontale 8 duquel est monté un moteur électrique 9 à courant continu dont l'arbre 10 est vertical, dirigé vers le haut et coaxial avec le godet 1. Un noyau en fer doux 11 coiffe à son extrémité inférieure l'arbre 10 du moteur 9 avec lequel il est coaxial et sur lequel il est serré par une vis 12. Ce noyau 11 traverse une bobine verticale 13 reliée à une source de courant continu non représentée et entourée par un blindage magnétique 13a. Le noyau 11 traverse ensuite un palier de guidage 14 monté dans la partie supérieure 15 du bâti 7, au-dessus duquel il fait saillie par un doigt 16 excentré par rapport à l'axe commun du godet 1, du moteur 9 et du noyau 11.

Un premier détecteur réflex 17, de type connu, est monté à l'extérieur du godet 1, mais à son voisinage immédiat, son axe étant horizontal et situé sensiblement au niveau de la bille 5. Un second détecteur réflex 18, similaire au premier, est monté à la hauteur du doigt excentré 16 qui surmonte le noyau tournant 11, et son axe est également horizontal. Ces détecteurs 17 et 18 sont avantageusement montés sur une plaquette de circuit imprimé 19 qui est fixée à son tour sur le bâti 7.

On comprend que l'amenée électrique continue à la bobine 13 aimante le noyau tournant 11 qui la traverse, et ceci transforme le doigt excentré 16 de ce dernier en un pôle magnétique qui, à son tour, entraîne en rotation la bille 5 le long de son chemin de roulement annulaire 4.

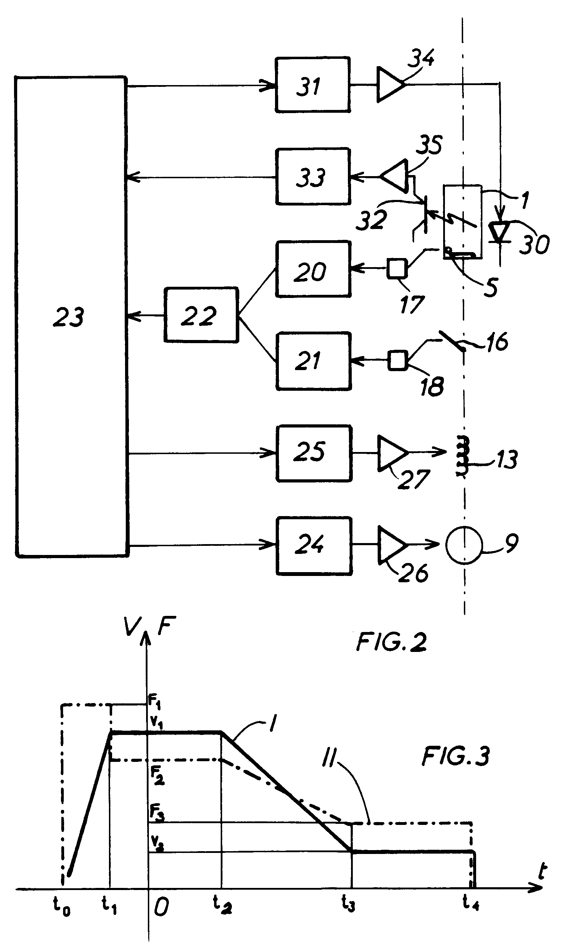

Lorsque du plasma a été versé dans le godet 1 de façon à recouvrir la bille 5, celle-ci tourne en synchronisme avec le doigt excentré 16, et les signaux électriques fournis par les deux détecteurs 17 et 18 coïncident exactement dans le temps. Au contraire, lorsque le plasma commence à se coaguler dans le godet 1 à la suite de l'introduction de réactifs appropriés, la bille 5 ralentit et les signaux de détecteur 17 associé à la bille 5, d'une part, et ceux du détecteur 18 correspondant au doigt d'entraînement 16, d'autre part, commencent à se décaler les uns par rapport aux autres. Cette information est détectée et exploitée par le dispositif électronique de la figure 2.

Sur le schéma de cette figure, on retrouve la bille 5 entraînée en rotation dans le godet 1 par le doigt excentré 16 sous l'influence du moteur 9 et de la bobine 13, ainsi que les détecteurs 17 et 18 associés respectivement à la bille 5 et au doigt tournant 16.

Les capteurs 17 et 18 sont reliés par l'intermédiaire de cellules de filtrage, 20 et 21 respectivement, à un compteur comparateur 22 dont les informations alimentent un microprocesseur 23. Ce dernier commande également l'alimentation en courant continu du moteur 9 et de la bobine 13 par l'intermédiaire de régulateurs 24 et 25, respectivement, et d'amplificateurs respectifs 26 et 27.

Ainsi, le comparateur 22 détecte le ralentissement de la bille 5 lorsque le plasma commence à se coaguler. Le microprocesseur 23 qui lui est associé exploite cette information, par exemple en affichant la valeur du temps de coagulation, en l'imprimant, en la mettant en mémoire, etc. De telles opérations sont bien connues des spécialistes en la matière, ainsi que les dispositifs qui sont nécessaires pour les mener à bonne fin, et ils ne seront donc pas décrits ici de manière plus détaillée.

D'autre part, et comme on l'a dit plus haut, il est avantageux d'imposer une loi de pilotage en fonction du temps à la vitesse de rotation et à la force d'entraînement de la bille 5, ce qui a lieu en agissant sur la tension aux bornes du moteur 9 et, respectivement, sur l'intensité du courant qui traverse la bobine 13. La valeur de ces grandeurs électriques à chaque instant est imposée par le microprocesseur 23 à travers les régulateurs 24, 25 et les amplificateurs 26, 27.

Cette loi de pilotage peut se traduire par les courbes de la figure 3 qui représentent, en fonction du temps t en secondes, les variations de la vitesse de rotation V du moteur 9, et donc de la bille 5, pour la courbe I, et celle de la force d'entraînement F de cette même bille 5 pour la courbe II.

Comme on le voit sur cette figure, en partant de l'instant du démarrage $t_0$, et jusqu'à un instant $t_1$ légèrement inférieur au début de l'essai qui correspond à l'origine O des coordonnées, la vitesse croit linéairement jusqu'à une valeur $V_1$ qui peut être égale à 10 tr/s, cependant que la force d'entraînement reste égale à une valeur relativement élevée $F_1$. La vitesse reste ensuite constante jusqu'à un temps $t_2$ postérieur, de 10 secondes par exemple, au début O de l'essai, cependant que la force reste également constante, mais après avoir pris une nouvelle valeur $F_2$ inférieure à la précédente. Les deux grandeurs en question décroissent alors linéairement jusqu'à un temps $t_3$ qui peut être égal à 60 secondes - comptées, bien entendu, à partir du début O de l'essai - pour rester constantes jusqu'au temps $t_4$ de fin de l'essai. Ce deuxième palier correspond à une vitesse $V_2$ qui peut être égale à 3 tr/s environ, et à une force $F_3$ égale environ à la moitié de $F_2$.

Ainsi qu'on l'a exposé plus haut, cette loi de pilotage permet d'obtenir une bonne précision, de l'ordre de 0,1 seconde, au début de la mesure, et une bonne sensibilité à la fin. Le dispositif selon l'invention autorise donc la détection de caillots forts, puis faibles, grâce à une variation progressive et commandée dans le temps des paramètres du système.

En revenant à la figure 1, on notera encore que le dispositif selon la présente invention peut comporter en outre un densitomètre optique destiné aux mesures dites de colorimétrie et monté sur le bloc de mesure 6 qui sert également de support pour le coagulomètre mécanique décrit ci-avant.

Ce densitomètre comprend essentiellement une source lumineuse constituée par une diode électroluminescente 30, un filtre optique passe-bande 31 et un récepteur optique constitué par une photodiode 32 suivie d'un filtre électronique. La diode 30 est montée sur une plaquette de circuit imprimé 30a fixée au bloc de mesure 6, cependant que le filtre 31 et la photodiode 32 le sont sur une autre plaquette de circuit imprimé 32a, également fixée au bloc de mesure 6. On retrouve ces éléments, représentés de manière schématique, sur la figure 2 à laquelle on se référera maintentant et sur laquelle le filtre électronique de la photodiode 32 est désigné par le repère 33.

Selon un exemple de réalisation particulier donné à titre d'exemple non limitatif, la diode électroluminescente 30, dont l'intensité lumineuse maximale se situe vers 480 nm, est alimentée à partir du microprocesseur 23 par un courant régulé et haché à quelques kilohertz, et ce, à travers le filtre 31 qui transmet la lumière jusqu'à 500 nm environ. Cette diode 30 éclaire la photodiode 32 avec laquelle elle est placée en ligne, de façon que le faisceau lumineux traverse le plasma et le godet 1 dans un plan horizontal au-dessus de la bille 5. Les informations fournies par la photodiode 32 sont transmise au microprocesseur 23 à travers le filtre 33 qui permet de ne pas retenir que le signal haché émis par la source 30. Enfin, des amplificateurs 34 et 35 sont interposés, respectivement, entre les filtres 31, 33 et les diodes 30, 32.

## Revendications

1. Dispositif pour mesurer le temps de modification de l'état physique d'un milieu fluide, et, en particulier, pour mesurer le temps de coagulation de sang, du genre comprenant un godet vertical qui contient le fluide et dans lequel sont introduits les réactifs nécessaires, tandis qu'y est placée une bille ferromagnétique entraînée en rotation, que le godet présente à sa partie inférieure un chemin de roulement annulaire pour ladite bille et que des moyens sont prévus pour détecter les modifications du mouvement de la bille qui résultent de la coagulation, caractérisé, en combinaison, en ce que la bille est directement entraînée en rotation par un champ magnétique tournant extérieur, et que les moyens de détection (17) + (18) sont agencés pour comparer les vitesses de rotation de la bille et du champ magnétique tournant, au moment de ladite modification d'état physique.

2. Dispositif selon la revendication 1, caractérisé par le fait que, pour entraîner en rotation ladite bille (5), il comprend un moteur électrique (9) dont l'arbre de sortie (10) est solidaire d'un noyau ferromagnétique (11) qui est disposé sous le godet (1) contenant la bille (5) en ayant le même axe que lui, qui traverse une bobine (13) alimentée en courant et qui se termine à sa partie supérieure par un doigt ferromagnétique (16) excentré par rapport à l'axe commun de ce noyau (11) et du godet (1), et situé immédiatement audessous de ce dernier (1).

3. Dispositif selon l'une quelconque des revendications 1 et 2, caractérisé par le fait qu'il comprend un premier détecteur réflex (17) associé à ladite bille (5), un second détecteur réflex (18) similaire au premier (17) et associé au doigt excentré (16) qui entraîne la bille (5), et un comparateur (22) relié à un microprocesseur (23) permettant de repérer tout défaut de synchronisme entre les signaux délivrés par lesdits deux détecteurs.

4. Dispositif selon la revendication 1, caractérisé par le fait que, pour entraîner en rotation ladite bille, il comprend une pluralité de pôles fixes qui entourent ledit godet (1) sensiblement sur le même plan que la bille (5) et qui sont magnétisés par des bobines alimentées par des courants variables de façon à produire un champ tournant dans le plan de la bille (5).

5. Dispositif selon l'une quelconque des revendications 1 à 4, caractérisé par le fait qu'il comporte des moyens pour imprimer à ladite bille (5) une vitesse de rotation et une force d'entraînement variables en fonction du test réalisé et de la durée de la mesure.

6. Dispositif selon la revendication 5, caractérisé par le fait que lesdits moyens pour imprimer à ladite bille (5) une vitesse de rotation et une force d'entraînement variables sont tels que les valeurs de ces grandeurs présentent au début de la mesure un palier où elles sont relativement élevées, et un autre palier à la fin de la mesure où ces valeurs sont plus faibles, ces deux paliers étant reliés par une diminution progressive.

7. Dispositif selon l'une quelconque des revendications 1 à 3, 5 et 6, caractérisé par le fait que ledit moteur (9) d'entraînement de la bille (5) est alimenté, ainsi que ladite bobine (13), par l'intermédiaire de moyens de régulation respectifs (24 à 27) commandés par un microprocesseur (23).

8. Dispositif selon l'une quelconque des revendications 1 à 7, caractérisé par le fait qu'il inclut un densitomètre optique comprenant une diode électroluminescente (30) qui est alimentée à partir d'un microprocesseur (23) et qui éclaire une photodiode (32) à travers un filtre optique (31), le faisceau lumineux passant à travers ledit godet (1), au-dessus dedite bille (5), et les informations de la photodiode (32) étant transmises audit microprocesseur (23).

9. Dispositif selon l'une quelconque des revendications 1 à 8 caractérisé par le fait qu'il comprend, dans un même bâti (2), une pluralité de godets (1) associés chacun à un ensemble de mesure (9, 13, 16, 17, 18) indépendant.

10. Dispositif selon l'une quelconque des revendications 1 à 9, caractérisé par le fait qu'il comporte un dispositif de régulation de la température, une alimentation électrique et/ou des interfaces homme/machine.

## Patentansprüche

1. Messvorrichtung für die Anderungszeit des physikalischen Zustandes eines flüssigen Mittels und insbesondere zur Messung der Blutgerinnungszeit, des Typs mit einem senkrechten, die Flüssigkeit enthaltenden, Becher, in den die nötigen Reagenzstoffe eingeführt werden und in dem eine ferromagnetische Kugel angeordnet ist, welche in Drehung versetzt wird und der Becher im unteren Teil eine ringförmige Laufspur für besagte Kugel aufweist und Mittel vorgesehen sind, um die sich aus der Gerinnung ergebenden Bewegungsänderungen der Kugel zu erfassen, in Kombination dadurch gekennzeichnet, dass die Kugel direkt durch ein äusseres magnetisches Drehfeld in Drehung versetzt wird und die Erfassungsmittel (17) und (18) so ausgelegt sind, dass sie die Drehgeschwindigkeiten der Kugel und des magnetischen Drehfeldes im Augenblick der besagten physikalischen Zustandsänderung vergleichen.

2. Vorrichtung nach Anspruch 1, dadurch gekennzeichnet, dass sie, um die besagte Kugel (5) in Drehung zu versetzen, einen Elektromotor (9) aufweist, dessen Abtriebswelle (10) fest mit einem ferromagnetischen Kern (11) verbunden ist, welcher in dem die Kugel (5) enthaltenden Becher (1) angeordnet ist, und die gleiche Achse hat, wie dieser und diese Achse eine mit Strom gespeiste Spule (13) durchquert und an ihrem oberen Teil in einem ferromagnetischen Finger (16) endet, der im Verhältnis zur gemeinsamen Achse des Kerns (11) und des Bechers (1) verschoben und direkt über dem letzteren angeordnet ist.

3. Vorrichtung nach einem der Ansprüche 1 und 2, dadurch gekennzeichnet, dass sie einen ersten, der besagten Kugel (5) zugeordneten, Reflexdetektor (17) aufweist, einen zweiten, dem ersten ähnlichen und dem Finger (16), welcher die Kugel in Bewegung versetzt, zugeordneten Reflexdetektor (18), und einen einem Mikroprozessor (23) zugeordneten Kompaprator (22), welcher es ermöglicht, Störungen im Synchronismus der von den besagten beiden Detektoren abgegebenen Signale zu erfassen.

4. Vorrichtung nach Anspruch 1, dadurch gekennzeichnet, dass sie, um besagte Kugel in Drehung zu versetzen, eine Vielzahl von stationären Polen aufweist, welche besagten Becher (1) etwa auf der gleichen Ebene wie die Kugel (5) umgeben und von mit variablen Strömen gespeisten Spulen magnetisiert werden, um auf der Ebene der Kugel (5) ein Drehfeld zu erzeugen.

5. Vorrichtung nach einem der Ansprüche 1 bis 4, dadurch gekennzeichnet, dass sie Mittel aufweist, um besagter Kugel (5) eine je nach dem ausgeführten Test und der Messdauer unterschiedliche Drehgeschwindigkeit mitzuteilen.

6. Vorrichtung nach Anspruch 5, dadurch gekennzeichnet, dass besagte Mittel, um besagter Kugel (5) eine unterschiedliche Drehgeschwindigkeit und Antriebskraft mitzuteilen, so ausgelegt sind, dass die Werte dieser Grössen zu Beginn der Messung eine Stufe aufweisen, wo sie relativ hoch sind und eine andere Stufe, am Ende der Messung, wo diese Werte geringer sind, und diese beiden Stufen durch eine Folge allmählich abnehmender Werte verbunden sind.

7. Vorrichtung nach einem der Ansprüche 1 bis 3, 5 und 6, dadurch gekennzeichnet, dass besagter Motor (9) zum Antrieb der Kugel (5) sowie die Spule (13) von entsprechenden von einem Mikroprozessor gesteuerten Reguliermitteln (24 bis 27) gespeist werden.

8. Vorrichtung nach einem der Ansprüche 1 bis 7, dadurch gekennzeichnet, dass sie einen optischen Densitometer aufweist, mit einer LED-Diode (30), die von einem Mikroprozessor (23) aus gespeist wird und eine Photodiode (32) durch einen optischen Filter (31) hindurch beleuchtet, wobei das Lichtbündel über besagter Kugel (5) den besagten Becher (1) durchquert und die Informationen der Photodiode (32) an besagten Mikroprozessor (23) übermittelt werden.

9. Vorrichtung nach einem der Ansprüche 1 bis 8, dadurch gekennzeichnet, dass sie, im gleichen Rahmen (2), eine Vielzahl von jeweils einer unabhängigen Messeinheit (9, 13, 16, 17, 18) zugeordneten Bechern (1) aufweist

10. Vorrichtung nach einem der Ansprüche 1 bis 9, dadurch gekennzeichnet, dass sie eine Temperaturregelvorrichtung, eine elektrische Stromquelle und,/oder Mensch/Maschinen-Interfacesyteme aufweist.

## Claims

1. A device for measuring the time of modification of the physical state of a fluid medium and, in particular, for measuring the coagulation time of blood, of the kind comprising a vertical cup which contains the fluid and in which the necessary reagents are introduced, while a ferromagnetic ball is placed there and is driven in rotation, the cup having in its lower part an annular travel path for said ball and means being provided for detecting the modifications of the movement of the ball resulting from coagualtion, characterized, in combination, in that the ball is directly rotated by an external rotating magnetic field and that the detection means (17) and (18) are provided for comparing the rotational speed of the ball and of the rotating magnetic field at the time of said modification of the physical state.

2. The device as claimed in claim 1, characterized in that, for driving said ball (5) in rotation, it comprises an electric motor (9) whose output shaft (10) is fast with a ferromagnetic core (11) which is disposed under the cup (1) containing the ball (5) while having the same axis as it, which passes through a coil (13) fed with current and which ends at its upper part in a ferromagnetic finger (16) off-centered with respect to the common axis of this core (11) and the cup (1), and situated immediately below the latter (1).

3. The device as claimed in one of claims 1 and 2, characterized in that it comprises a first reflex detector (17) associated with the said ball (5), a second reflex detector (18) similar to the first one (17) and associated with the off-centered finger (16) which drives the ball (5), and a comparator (22) connected to a microprocessor (23) for detecting any synchronism defect between the signals delivered by said two detectors.

4. The device as claimed in claim 1, characterized in that, for driving said ball in rotation, it comprises a plurality of fixed poles which surround said cup (1) substantially in the same plane as the ball (5) and which are magnetized by coils fed with variable current so as to produce a rotating field in the plane of the ball (5).

5. The device as claimed in one of claims 1 to 4, characterized in that it comprises means for imparting to said ball (5) a rotational speed and a drive force which are variable as a function of the test carried out and of the duration of the measurement.

6. The device as claimed in claim 5, characterized in that said means for imparting a variable speed of rotation and a variable drive force to said ball (5) are such that the values of these magnitudes have at the beginning of the measurement a level stretch where they are relatively bigh and another level stretch at the end of the measurement where these values are lower, these two stretches being connected together by a progressive decrease.

7. The device as claimed in one of claims 1 to 3, 5 and 6, characterized in that said motor (9) for driving the ball (5) is supplied, as well as said coil (13), through respective regulation means (24 to 27) controlled by a microprocessor (23).

8. The device as claimed in one of claims 1 to 7, characterized by an optical densitometer with a LED (30) which is fed from a microprocessor (23) and which illuminates a photodiode (32) through an optical filter (31), the light beam passing through said cup (1) above said ball (5), and the information from the photodiode (32) being transmitted to said microprocessor (23).

9. The device as claimed in one of claims 1 to 8, characterized in that it comprises, in the same frame (2), a plurality of cups (1), each associated with an independent measurement assembly ( 9, 13, 16, 17, 18 ).

10. The device as claimed in one of claims 1 to 9, characterized in that it comprises a temperature regulation device, an electric supply source and/ or man-/machine interfaces.

FIG.1

FIG.2

FIG.3